# EUROPEAN PATENT APPLICATION

(11) **EP 3 842 521 A1**
(43) Date of publication of application: **30.06.2021**
(21) Application number: 19852222.9
(22) Date of filing: 15.08.2019
(51) Int. Cl.: C12N 5/074, C12M 3/00

(54) **CELL CULTURE METHOD AND CELL CULTURE APPARATUS**

(30) Priority: 22.08.2018 JP 2018155328
(71) Applicant: Nikkiso Co., Ltd., Tokyo 150-6022 (JP)
(72) Inventor: KITAGAWA, Fumihiko, Ishikawa 920-0177 (JP)
(74) Representative: Algemeen Octrooi- en Merkenbureau B.V.
(86) International application number: PCT/JP2019/032069
(87) International publication number: WO 2020/040041

(57) **Abstract**

This cell culture method comprises a step for culturing cells 8 in a culture medium 10 that satisfies at least one among the following condition (I) and condition (II). Condition (I): the concentration of a lactic acid in the culture medium is less than 7.5 mM. Condition (II): the concentration of an ammonia in the culture medium is less than 2.5 mM.

## Description

### [TECHNICAL FIELD]

The present invention relates to a cell culture method and a cell culture apparatus.

### [BACKGROUND ART]

In recent years, in fields such as pharmaceutical manufacturing and regenerative medicine, it has been required to artificially and efficiently mass-culture cells and microorganisms. Examples of cells for which mass culture is required include antibody-producing cells such as Chinese hamster ovary cells (CHO cells); and pluripotent stem cells such as embryonic stem cells (ES cells) and induced pluripotent stem cells (iPS cells). These cells, if stably cultured in large quantities for a long period of time, make it possible to efficiently produce biological substances such as monoclonal antibodies, and differentiation-inducing tissues derived from pluripotent stem cells.

A possible way of industrial mass production of cells may be suspension stirred culture with use of a culture tank such as a spinner flask. The suspension stirred culture, however, tends to need large scale of equipment. Increase of cell density during culture will therefore be effective for cost reduction. Increase of the cell density has, however, been known to suppress proliferation of cells. This is because concentration levels of wastes (metabolites) in the culture solution (liquid medium) will increase due to densification of cells, and thereby the proliferative activity of cells will decline. Lactic acid and ammonia are known as representative wastes that can affect the cells.

It is therefore preferred to remove lactic acid and ammonia accumulated in the culture solution, in order to allow the cells to stably proliferate in a dense state. On the other hand, Patent Literature 1 for example discloses a cell culture apparatus in which a cell culture tank and a component adjusting solution tank are connected by a liquid feeding line provided with a culture solution component adjustment membrane that allows components to permeate depending on concentration difference. In this cell culture device, the waste products accumulated in the culture solution move to the component adjusting solution side, so that the concentration in the culture solution decreases. At the same time, the nutrients whose concentration has decreased during the culture are transferred from the component adjusting solution to the culture solution, and are replenished. The environment in the culture solution is thus maintained in a condition suitable for cell culture.

### [Patent Literature]

[Patent Literature 1] WO2015/122528

### [SUMMARY OF INVENTION]

### [TECHNICAL PROBLEM]

The cell culture apparatus disclosed in Patent Literature 1 has removed waste products from the culture solution by use of the principle of dialysis. In order to attain sufficient removal of waste products, the capacity of the component adjusting solution tank has therefore been set to 10 times or more the capacity of the cell culture tank. In addition, the concentration levels of lactic acid and ammonia in the culture solution have not been monitored. That is, there have been no extensive investigations on timing, volume and so forth of the culture solution fed from the cell culture tank to the ingredient conditioning solution tank, in view of regulating the lactic acid concentration and the ammonia concentration. There is therefore a problem that the required liquid amounts huge and becomes costly. In particular, in a case where the culture solution itself is used as the component adjusting solution, a large amount of expensive medium is consumed, which further increases the cost.

The present invention was conceived in consideration of such circumstances, wherein one object of which is to provide a technique for mass-culture of cells at low cost.

### [SOLUTION TO PROBLEM]

Aimed at solving the aforementioned problem, one aspect of the present invention relates to a cell culture method. The cell culture method includes culturing a cell in a culture solution that satisfies at least one of condition (I) or condition (II) below.
Condition (I): lactic acid concentration in the culture solution is lower than 7.5 mM.
Condition (II): ammonia concentration in the culture solution is lower than 2.5 mM.

This aspect enables mass-culture of a cell at low cost.

In this aspect, the cell may be a pluripotent stem cell. The lactic acid concentration in condition (I) may be 5 mM or lower. The ammonia concentration in condition (II) may be 1 mM or lower.

Another aspect of the present invention relates to a cell culture apparatus. The cell culture apparatus includes a culture vessel structured to house a cell and a culture solution; and a conditioner structured to condition the culture solution so as to satisfy at least one of condition (I) or condition (II) below.
Condition (I): lactic acid concentration in the culture solution is lower than 7.5 mM.
Condition (II): ammonia concentration in the culture solution is lower than 2.5 mM.

The conditioner in this aspect may include: a storage tank structured to store a replenishing solution; a feed pump structured to fees the replenishing solution from the storage tank to the culture vessel; a concentration sensor structured to detect at least one of lactic acid concentration or ammonia concentration in the culture solution; and a controller structured to drive the feed pump in response to result of detection made by the concentration sensor. The conditioner may include: a substance exchanger structured to house a substance exchange membrane and an ingredient conditioning solution, and, upon feeding of the culture solution, allows substance exchange to proceed through the substance exchange membrane between the ingredient conditioning solution and the culture solution; a circulation pump structured to circulate the culture solution between the culture vessel and the substance exchanger; a concentration sensor structured to detect at least one of lactic acid concentration or ammonia concentration in the culture solution; and a controller structured to drive the circulation pump in response to result of detection made by the concentration sensor. The conditioner may have an adsorbent structured to adsorb at least one of lactic acid or ammonia in the culture solution.

Note that also free combinations of these constituents, and also any of the constituents and expressions exchanged among the method, device and system, are valid as the aspects of the present invention.

### [ADVANTAGEOUS EFFECTS OF INVENTION]

The present invention enables mass-culture of a cell at low cost.

### [BRIEF DESCRIPTION OF DRAWINGS]

Fig. 1 is a schematic drawing illustrating a cell culture apparatus of Embodiment 1.
Fig. 2 is a schematic drawing illustrating a cell culture apparatus of Embodiment 2.
Fig. 3 is a schematic drawing illustrating a cell culture apparatus of Embodiment 3.
Fig. 4 is a schematic drawing illustrating a cell culture apparatus of Embodiment 4.
Fig. 5 is a graph illustrating relation between lactic acid concentration and cell density.
Fig. 6 presents optical microphotographs of cells cultured in a lactic acid-added medium.
Fig. 7 is a chart summarizing relation between lactic acid concentration and gene expression levels.
Fig. 8 is a graph illustrating relation between ammonia concentration and cell density.
Fig. 9 presents optical microphotographs of cells cultured in an ammonia-added medium.
Fig. 10 is a chart summarizing relation between ammonia concentration and gene expression levels.

### [DESCRIPTION OF EMBODIMENTS]

The present invention will be explained below on the basis of preferred embodiments, referring to the attached drawings. The embodiments are merely illustrative, and are not restrictive about the invention. All features and combinations thereof described in the embodiments are not always necessarily essential to the present invention. All constituents, members and processes illustrated in the individual drawings will be given same reference numerals, so as to properly avoid redundant explanations. Reduced scales and shapes of the individual parts in the individual drawings are properly given for simplicity of explanation, and should not be interpreted restrictively unless otherwise specifically noted. Also note that ordinal terms "first", "second" and so on used in this patent specification and in claims do not represent any sequential order or importance, and are used only for discrimination of one structure from the other. Further, in each drawing, some of the members that are not important for explaining the embodiment will be omitted.

The present inventors went through extensive investigations on effects of lactic acid and ammonia, which are representative wastes, exerted on cells, and found lactic acid concentration and ammonia concentration at which cells are adversely affected. The present inventors also found from our further investigations that lactic acid and ammonia can affect each state of proliferation and undifferentiation of the cell, at different concentration levels, when the cell is a pluripotent stem cell.

### Embodiment 1

The cell culture method according to this embodiment includes culturing a cell in a culture solution that satisfies at least one of condition (I) or condition (II) below.
Condition (I): lactic acid concentration in the culture solution is lower than 7.5 mM (7.5 × 10⁻³ mol/L).
Condition (II): ammonia concentration in the culture solution is lower than 2.5 mM.

The cell proliferation may be suppressed from being inhibited, when the culture solution contains lactic acid, with the lactic acid concentration kept lower than 7.5 mM. The cell proliferation may also be suppressed from being inhibited by lowered pH of the culture solution due to lactic acid. The cell may be retained in an undifferentiated state, when the lactic acid concentration is kept lower than 10 mM. Hence, the cell when cultured in the culture solution that satisfies condition (I) may be less affected by lactic acid, in both states of cell proliferation and undifferentiation.

The cell proliferation may be suppressed from being inhibited, when the culture solution contains ammonia, with the ammonia concentration kept lower than 10 mM. Meanwhile, the cell may be kept in the undifferentiation state, with the ammonia concentration kept lower than 2.5 mM. Hence, the cell when cultured in the culture solution that satisfies condition (II) may be less affected by ammonia, in both states of cell proliferation and undifferentiation.

The lactic acid concentration in condition (I) is preferably 5 mM or lower. This enables to further suppress the cell proliferation from being inhibited by lactic acid. Meanwhile, the ammonia concentration in condition (II) may be 1 mM or lower. This enables to further retain the cell in the undifferentiated state.

The cell possibly cultured in the culture solution is exemplified by pluripotent stem cells such as human iPS cell, human ES cell, and human Muse cell; somatic stem cells such as mesenchymal stem cell (MSC) and nephron progenitor cell; tissue cells such as human renal proximal tubule epithelial cell, human distal tubule epithelial cell, and human collecting duct epithelial cell; antibody-producing cell lines such as human fetal renal cell (HEK293 cell); and antibody-producing cell lines derived from animals other than human such as Chinese hamster ovary cell (CHO cell) and insect cell (SF9 cell). The cell is preferably the pluripotent stem cell.

The cell is preferably cultured by suspension culture. As the culture vessel for suspension culture, employable is a spinner flask, large stainless steel culture tank, cell culture bottle, cell culture bag and so forth. The culture solution is properly selectable depending on cells to be cultured.

### Cell Culture Apparatus

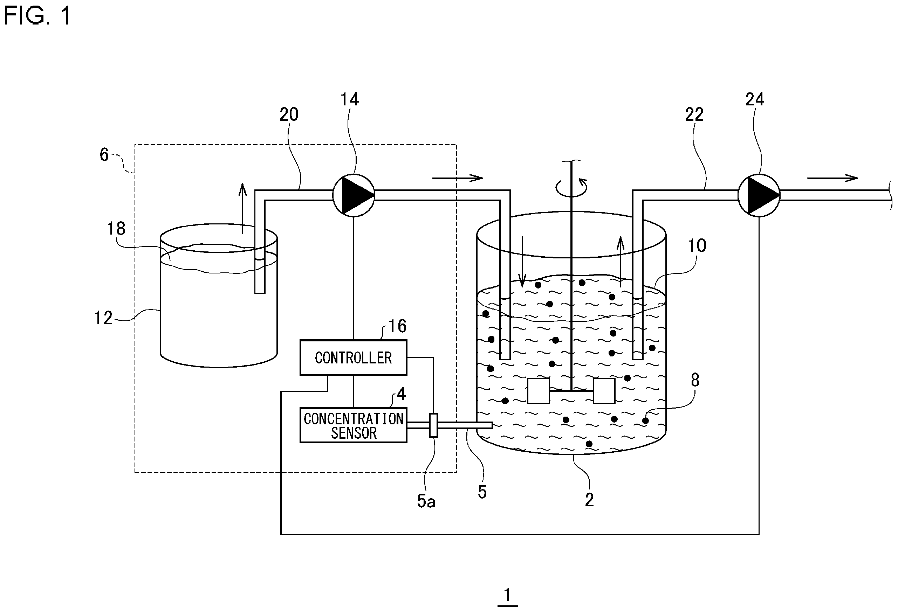

Next, the cell culture apparatus according to this embodiment will be explained. Fig. 1 is a schematic drawing illustrating a cell culture apparatus of Embodiment 1. A cell culture apparatus 1 has a culture vessel 2 and a conditioner 6, as the principal components. The culture vessel 2 is a bioreactor that houses a cell 8 and a culture solution 10. The conditioner 6 conditions the culture solution 10, so as to satisfy at least one of the aforementioned condition (I) and condition (II). The conditioner 6 of this embodiment has a storage tank 12, a feed pump 14, a concentration sensor 4, and a controller 16.

The storage tank 12 stores a replenishing solution 18. The replenishing solution 18 may be same as the culture solution 10 in the culture vessel 2, or may be high-concentration culture solution densified from the culture solution 10. The replenishing solution 18 may be a solution other than the medium, whose ingredients are blended depending on types of the cell 8.

The storage tank 12 and the culture vessel 2 are connected with a feed pipe 20. A feed pump 14 is connected in the middle of the feed pipe 20. The feed pump 14 may be any of known pumps such as Peristaltic pump, diaphragm pump or the like.

The concentration sensor 4 detects at least one of lactic acid concentration or ammonia concentration in the culture solution 10. As the concentration sensor 4, employable is any of known sensors such as a medium ingredient analyzer or the like. The lactic acid concentration and the ammonia concentration are detectable by employing any of known methods, such as colorimetry with use of predetermined measurement reagent, enzyme electrode method utilizing substrate specificity of enzyme, high performance liquid chromatography (HPLC), and so forth. The concentration sensor 4 and the culture vessel 2 are connected with a sampling tube 5. The culture solution 10 in the culture vessel 2 is sampled through the sampling tube 5 into the concentration sensor 4. The sampling tube 5 has, at the end connected to the culture vessel 2, an unillustrated filter attached thereto. This successfully suppresses the cell 8 from being sucked into the sampling tube 5. The concentration sensor 4 samples the culture solution 10 at regular temporal intervals for ingredient analysis. The sampling tube 5 is typically provided with a solenoid valve 5a. With open/close operation of the solenoid valve 5a controlled by the controller 16, flow of the culture solution 10 into the concentration sensor 4 is switched between allowed and blocked. This enables the concentration sensor 4 to repeat the ingredient analysis of the culture solution 10 in a certain timing.

The controller 16 drives the feed pump 14 in response to result of detection made by the concentration sensor 4. More specifically, the feed pump 14 is controlled so as to satisfy condition (I), when the concentration sensor 4 detects the lactic acid concentration. The feed pump 14 is controlled so as to satisfy condition (II), when the concentration sensor 4 detects the ammonia concentration. The feed pump 14 is controlled so as to satisfy condition (I) and condition (II), when the concentration sensor 4 detects both concentration levels. Note that the controller 16 may control the feed pump 14 so as to satisfy only one of conditions, even when the concentration sensor 4 is used for detecting both concentration levels.

The controller 16 in terms of hardware configuration is embodied by computer elements and circuits represented by CPU and memory, and the one in terms of software configuration is embodied by computer program product and so forth, all of which being properly depicted in Fig. 1 as functional blocks embodied by cooperation of these components. Those skilled in the art will easily understand that these functional blocks may be embodied in various ways, depending on combinations of the hardware and the software.

When the feed pump 14 is driven by the controller 16, the replenishing solution 18 is fed from the storage tank 12 through the feed pipe 20 to the culture vessel 2. The replenishing solution 18 in the storage tank 12 is thus filled in the culture vessel 2. The culture solution 10 in the culture vessel 2 has accumulated therein lactic acid and ammonia excreted from the cell 8. Meanwhile, the replenishing solution 18 has the lactic acid concentration and the ammonia concentration lower than those of the culture solution 10. The replenishing solution 18 is preferably free of lactic acid and ammonia. Hence, upon addition of the replenishing solution 18 to the culture solution 10, the lactic acid concentration and the ammonia concentration in the culture solution 10 decrease. Also medium ingredients such as glucose and protein, necessary for culturing the cell 8, are replenished to the culture solution 10.

The controller 16 puts the feed pump 14 under feedback control in response to result of detection made by the concentration sensor 4, so that the culture solution 10 satisfies condition (I) and/or condition (II). More specifically, the controller 16 accepts result of detection made by the concentration sensor 4, repetitively at certain times. When conditioning the culture solution 10 so as to satisfy condition (I), the controller 16 determines whether the lactic acid concentration in the culture solution 10 is kept lower than 7.5 mM or not, on the basis of result of detection made by the concentration sensor 4. If the lactic acid concentration is found to be 7.5 mM or higher, the controller 16 drives the feed pump 14. Upon falling of the lactic acid concentration below 7.5 mM, the feed pump 14 is stopped operating.

A concentration range is preferably preset with a certain margin added to 7.5 mM, which is a threshold value. The controller 16 drives the feed pump 14 if the lactic acid concentration exceeds the concentration range, meanwhile stops the feed pump 14 if the concentration falls below the concentration range. The concentration range is more preferably determined so as to have an upper limit value of 7.5 mM. That is, the controller 16 stops the feed pump 14, when the lactic acid concentration falls down to a level lower just by a predetermined value than 7.5 mM. Note that the upper limit value of the concentration range may alternatively be determined to a level lower than 7.5 mM, so as to more reliably suppress the lactic acid concentration in the culture solution 10 from reaching 7.5 mM and above.

When conditioning the culture solution 10 so as to satisfy condition (II), the controller 16 determines whether the ammonia concentration in the culture solution 10 is kept lower than 2.5 mM or not, in response to result of detection made by the concentration sensor 4. If the ammonia concentration is found to be 2.5 mM or higher, the controller 16 drives the feed pump 14. Upon falling of the ammonia concentration below 2.5 mM, the feed pump 14 is stopped operating.

A concentration range is preferably preset with a certain margin added to 2.5 mM, which is a threshold value. The controller 16 drives the feed pump 14 if the lactic acid concentration exceeds the concentration range, meanwhile stops the feed pump 14 if the concentration falls below the concentration range. The concentration range is more preferably determined so as to have an upper limit value of 2.5 mM. That is, the controller 16 stops the feed pump 14, when the ammonia concentration falls down to a level lower just by a predetermined value than 2.5 mM. Note that the upper limit value of the concentration range may alternatively be determined to a level lower than 2.5 mM, so as to more reliably suppress the ammonia concentration in the culture solution 10 from reaching 2.5 mM and above.

In a case where the culture solution 10 is conditioned so as to satisfy both of condition (I) and condition (II), the controller 16 stops the feed pump 14 if both conditions are satisfied, meanwhile drives the feed pump 14 if either or both conditions are not satisfied.

The culture vessel 2 is connected through a drain pipe 22 to an unillustrated waste tank. In the middle of the drain pipe 22, connected is a drain pump 24. The drain pump 24 employable here may be any of known pumps, just like the feed pump 14. The controller 16 also controls drive of the drain pump 24. The controller 16 drives the drain pump 24, accompanied by drive of the feed pump 14. With the drain pump 24 thus driven, the culture solution 10 in the culture vessel 2 is fed through the drain pipe 22 to the waste tank. This makes it possible to keep liquid level in the culture vessel 2 at constant. The drain pipe 22 is provided, at the end thereof connected to the culture vessel 2, with an unillustrated filter. This successfully suppresses the cell 8 from being sucked into the drain pipe 22. The drain pipe 22 may also be used as the sampling tube 5. In this case, the concentration sensor 4 samples the culture solution 10 through the drain pipe 22. Note that the drain pump 24 may be driven independently from the feed pump 14.

As explained above, the cell culture method of this embodiment includes culturing the cell in the culture solution 10 that satisfies at least one of condition (I) or condition (II) below.
Condition (I): lactic acid concentration in the culture solution 10 is lower than 7.5 mM.
Condition (II): ammonia concentration in the culture solution 10 is lower than 2.5 mM.

Moreover, the cell culture apparatus 1 of this embodiment includes the culture vessel 2 that houses the cell 8 and the culture solution 10; and the conditioner 6 that conditions the culture solution 10 so as to satisfy at least one of the aforementioned condition (I) or condition (II).

With the culture solution 10 conditioned so as to satisfy at least one of condition (I) or condition (II), it becomes possible to moderate any adverse effect possibly exerted by at least one of lactic acid or ammonia. This enables mass-culture of the cell 8 at high density. Alternatively, with the culture solution 10 conditioned so as to satisfy both of condition (I) and condition (II), it becomes possible to moderate any adverse effect possibly exerted by both of lactic acid and ammonia. This enables mass-culture of the cell 8 at high density in a more reliable manner.

Also note this embodiment specifies the concentration of each of lactic acid and ammonia, at which the cell 8 may be affected, and conditions the culture solution 10 by monitoring the concentration values of lactic acid and ammonia. This successfully reduces consumption of the ingredient conditioning solution and the culture solution, as compared with a known cell culture apparatus in which the culture solution is dialyzed to an unnecessarily excessive degree. This successfully enables cost reduction. This embodiment therefore enables mass-culture of the cell 8 at low cost.

In a case where the cell 8 is a pluripotent stem cell, reduction of lactic acid concentration and/or ammonia concentration enables not only high-density, mass-culture of the cell 8, but also enables the cell 8 to retain an undifferentiated state, that is, the cell 8 can remain pluripotent (can keep pluripotency). This therefore enables to obtain a large amount of the cell 8 suitable for producing differentiation-inducing tissues. Since a cell population with a uniform state is thus obtainable, and the efficiency of differentiation induction can be thus suppressed from degrading, so that costs for production of cellular medicine and regenerative medicine may be reduced.

In the cell culture apparatus 1 of this embodiment, the conditioner 6 has a storage tank 12 that stores a replenishing solution 18; the feed pump 14 that feeds the replenishing solution 18 from the storage tank 12 to the culture vessel 2; a concentration sensor 4 that detects at least one of lactic acid concentration or ammonia concentration in the culture solution 10; and a controller 16 that drives the feed pump 14 in response to result of detection made by the concentration sensor 4. The lactic acid concentration and the ammonia concentration in the culture solution 10 may be decreased by adding the replenishing solution 18 to the culture solution 10. The controller 16 also drives the drain pump 24 together with the feed pump 14, to discharge the culture solution 10 from the culture vessel 2. That is, this embodiment suppresses the waste concentration from increasing, by employing continuous culture in which an old medium is removed while replenishing a new medium.

### Embodiment 2

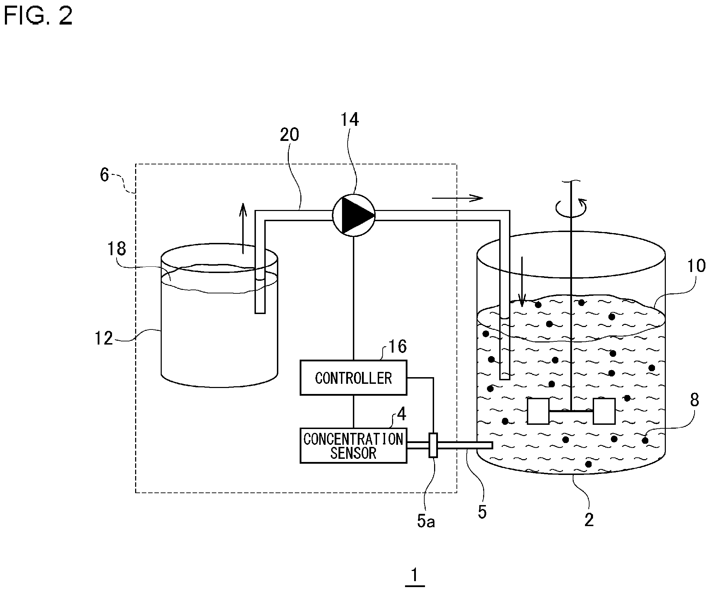

Embodiment 2 has a common structure with Embodiment 1, except for structures of the cell culture apparatus. The explanation below will deal with this embodiment, emphasizing the structures different from those in Embodiment 1, while briefing or skipping explanation of the common structure. Fig. 2 is a schematic drawing illustrating a cell culture apparatus of Embodiment 2.

The cell culture apparatus 1 of this embodiment has the culture vessel 2 and the conditioner 6, as the principal components. The cell 8 and the culture solution 10 are housed in the culture vessel 2. The conditioner 6 conditions the culture solution 10, so as to satisfy at least one of the aforementioned condition (I) and condition (II). The conditioner 6 of this embodiment has a storage tank 12, a feed pump 14, a concentration sensor 4, and a controller 16.

The storage tank 12 stores a replenishing solution 18. The storage tank 12 and the culture vessel 2 are connected with a feed pipe 20. A feed pump 14 is connected in the middle of the feed pipe 20. The concentration sensor 4 detects at least one of lactic acid concentration or ammonia concentration in the culture solution 10. The controller 16 drives the feed pump 14 in response to result of detection made by the concentration sensor 4. When the feed pump 14 is driven by the controller 16, the replenishing solution 18 is fed from the storage tank 12 through the feed pipe 20 to the culture vessel 2. The replenishing solution 18 in the storage tank 12 is thus filled in the culture vessel 2. Timing of driving of the feed pump 14 by the controller 16 is same as in Embodiment 1.

Unlike Embodiment 1, the cell culture apparatus 1 of this embodiment has neither the drain pipe 22 nor the drain pump 24. The volume of liquid in the culture vessel 2 therefore gradually increases. That is, this embodiment suppresses the waste concentration from elevating, by employing fed-batch culture in which the replenishing solution 18 is continuously fed to the culture vessel 2. In this structure, both of the number of cells and the volume of the culture solution in the culture vessel 2 increase with the elapse of culture period. Cell density in the culture solution 10 may thus be kept constant. Note that the size (capacity) of the culture vessel 2 is determined considering typically the culture period of the cell 8, the volume of the culture solution 10 housed in the culture vessel 2 at the start of culture, and the amount of addition of the replenishing solution 18. Also this embodiment can demonstrate effects same as in Embodiment 1.

### Embodiment 3

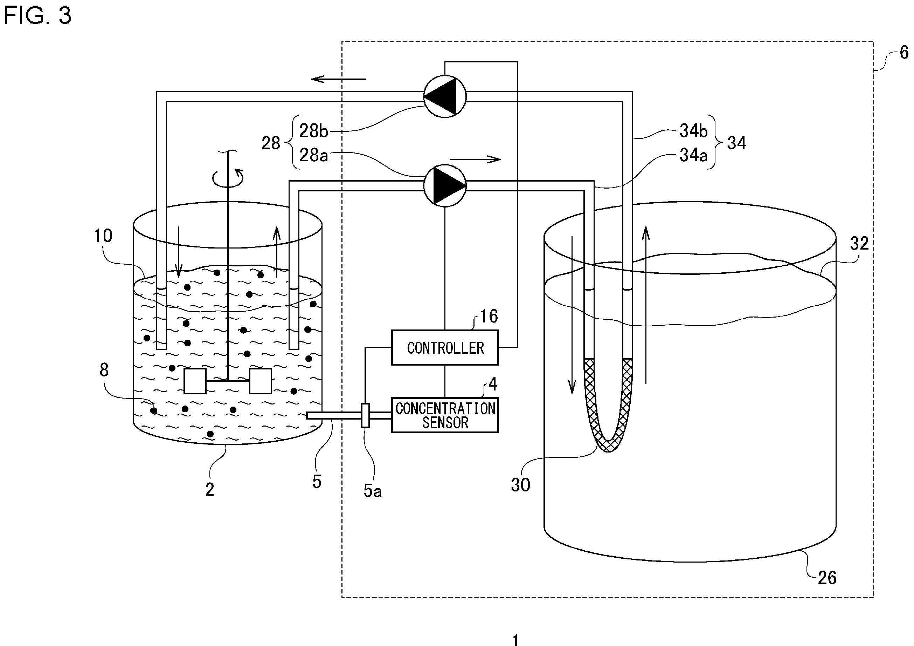

Embodiment 3 has a common structure with Embodiment 1, except for the structure of the cell culture apparatus. The explanation below will deal with this embodiment, emphasizing the structures different from those in Embodiment 1, while briefing or skipping explanation of the common structure. Fig. 3 is a schematic drawing illustrating a cell culture apparatus of Embodiment 3.

The cell culture apparatus 1 of this embodiment has the culture vessel 2 and the conditioner 6, as the principal components. The cell 8 and the culture solution 10 are housed in the culture vessel 2. The conditioner 6 conditions the culture solution 10, so as to satisfy at least one of the aforementioned condition (I) or condition (II). The conditioner 6 of this embodiment has a substance exchanger 26, a circulation pump 28, the concentration sensor 4, and the controller 16.

The substance exchanger 26 houses a substance exchange membrane 30 and an ingredient conditioning solution 32. Upon being fed with the culture solution 10 in the culture vessel 2, the substance exchanger 26 takes part in substance exchange through the substance exchange membrane 30, between the ingredient conditioning solution 32 and the culture solution 10. The substance exchange membrane 30 may employ any of known substance exchange membranes made of hollow fiber or the like. Like the replenishing solution 18, the ingredient conditioning solution 32 may be same as the culture solution 10 in the culture vessel 2, or may be high-concentration culture solution condensed from the culture solution 10. Alternatively, any solution other than the medium, whose ingredients are blended depending on types of the cell 8, is employable.

The culture vessel 2 and the substance exchanger 26 are connected with a circulation path 34. The circulation path 34 includes a forward path 34a having one end connected to the culture vessel 2 and the other end connected to the substance exchanger 26, and a return path 34b having one end connected to the substance exchanger 26 and the other end connected to the culture vessel 2. The ends of the forward path 34a and the return path 34b, on the side closer to the substance exchanger 26, are connected through the substance exchange membrane 30. The substance exchange membrane 30 is immersed in the ingredient conditioning solution 32. Hence a flow path of the culture solution 10, composed of the forward path 34a, the substance exchange membrane 30 and the return path 34b, is provided so as to bridge the culture vessel 2 and the substance exchanger 26.

The circulation pump 28 circulates the culture solution 10 between the culture vessel 2 and the substance exchanger 26. More specifically, the circulation pump 28 has a first pump 28a and a second pump 28b. The first pump 28a is connected in the middle of the forward path 34a, and feeds the culture solution 10 from the culture vessel 2 to the substance exchange membrane 30 of the substance exchanger 26. The second pump 28b is connected in the middle of the return path 34b, and feeds the culture solution 10 from the substance exchange membrane 30 to the culture vessel 2.

The concentration sensor 4 detects at least one of lactic acid concentration or ammonia concentration in the culture solution 10. The controller 16 drives the circulation pump 28 in response to result of detection made by the concentration sensor 4. Upon start of driving of the circulation pump 28 by the controller 16, the culture solution 10 circulates through the circulation path 34, between the culture vessel 2 and the substance exchanger 26. During the process, substance exchange proceeds through the substance exchange membrane 30, between the culture solution 10 and the ingredient conditioning solution 32. As a consequence, lactic acid and ammonia in the culture solution 10 move to the ingredient conditioning solution 32, meanwhile medium ingredients such as glucose and protein in the ingredient conditioning solution 32 move to the culture solution 10. Timing of driving of the circulation pump 28 by the controller 16 is same as the timing of driving of the feed pump 14 in Embodiment 1.

The lactic acid concentration and ammonia concentration in the culture solution 10 thus decrease. Also medium ingredients such as glucose and protein, necessary for culturing the cell 8, are replenished to the culture solution 10. Note that unillustrated filter is provided at the end of the forward path 34a on the side connected to the culture vessel 2. This successfully suppresses the cell 8 from being sucked into the drain pipe 22. The substance exchanger 26 may have an adsorbent for waste placed therein. This successfully lowers the concentration of the waste dissolved in the ingredient conditioning solution 32, and reduces the amount of ingredient conditioning solution 32 necessary for the substance exchange. The adsorbent employable here may be any of known adsorbents such as silica, activated carbon and so forth, capable of adsorbing at least one of lactic acid or ammonia.

That is, this embodiment suppresses the waste concentration in the culture solution 10 from elevating, by allowing the culture solution 10 to flow from the culture vessel 2 through the substance exchange membrane 30 in the substance exchanger 26 to perform substance exchange, and then by discharging the waste into the ingredient conditioning solution 32 in the substance exchanger 26. Also this embodiment can demonstrate effects same as in Embodiment 1.

### Embodiment 4

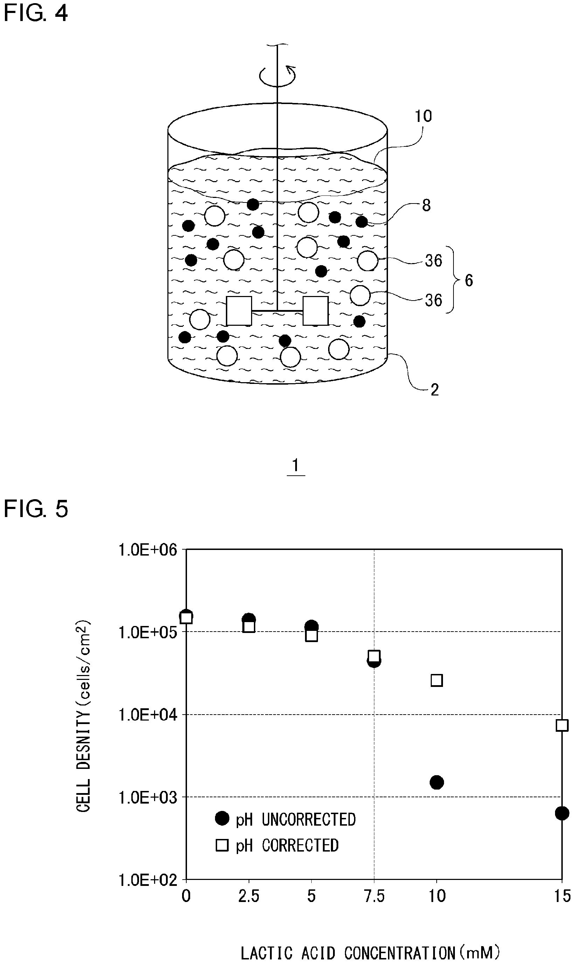

Embodiment 4 has a common structure with Embodiment 1, except for the structure of the cell culture apparatus. The explanation below will deal with this embodiment, emphasizing the structures different from those in Embodiment 1, while briefing or skipping explanation of the common structure. Fig. 4 is a schematic drawing illustrating a cell culture apparatus of Embodiment 4.

The cell culture apparatus 1 of this embodiment has the culture vessel 2 and the conditioner 6. The cell 8 and the culture solution 10 are housed in the culture vessel 2. The conditioner 6 conditions the culture solution 10, so as to satisfy at least one of the aforementioned condition (I) or condition (II). The conditioner 6 in this embodiment has an adsorbent 36.

The adsorbent 36 employable here may be any of known adsorbents such as silica, activated carbon and so forth. The adsorbent 36 may be a single adsorbent that adsorbs both of lactic acid and ammonia, or may be a mixture of a first adsorbent that adsorbs lactic acid and a second adsorbent that adsorbs ammonia. The culture solution 10 is conditioned so as to satisfy at least one of condition (I) or condition (II) described previously, by bringing the culture solution 10 into contact with the adsorbent 36. Amount of the adsorbent 36 necessary for satisfying condition (I) and/or condition (II) may be properly determined by a skilled person in the art. The adsorbent 36 preferably chosen here is less toxic to the cell 8.

The adsorbent 36 is typically granular, and is housed in the culture vessel 2. The adsorbent 36 disperses, sediments or suspends in the culture solution 10, in the culture vessel 2. The adsorbent 36 in this case preferably has a predetermined value or larger size, which is typically 10 µm or larger, in view of preventing phagocytosis by the cells 8. Upon bringing the culture solution 10 into contact with the adsorbent 36, lactic acid and/or ammonia in the culture solution 10 are adsorbed to the adsorbent 36. The lactic acid concentration and/or the ammonia concentration in the culture solution 10 may thus be reduced, and thereby condition (I) and/or condition (II) may be satisfied. Hence, also this embodiment can demonstrate effects same as in Embodiment 1.

The conditioner 6 may alternatively have a structure in which the adsorbent 36 is packed in an unillustrated adsorption column connected to the culture vessel 2. The culture solution 10 in the culture vessel 2 in this case is fed to the adsorption column, and is brought into contact with the adsorbent 36. The conditioner 6 may alternatively have a structure in which the adsorbent 36 is supported on the inner wall of the culture vessel 2. Methods for supporting the adsorbent 36 on the inner wall of the culture vessel 2 are exemplified by a method of adhering the adsorbent 36 to the inner wall of the culture vessel 2; and, when the culture vessel 2 is made of a resin, a method of molding the culture vessel 2 by using the resin preliminarily mixed with the adsorbent 36.

Again alternatively, the conditioner 6 may have a structure in which the inside of the culture vessel 2 is partitioned with a diaphragm typically made of a porous membrane, into an upper compartment and a lower compartment, with the adsorbent 36 housed in the lower compartment. The cell 8 in this structure is housed in the upper compartment. The culture solution 10 can move through the diaphragm between the upper compartment and the lower compartment. In contrast, the cell 8 and the adsorbent 36 cannot pass through the diaphragm. Upon bringing the culture solution 10 into contact with the adsorbent 36 housed in the lower compartment, lactic acid and/or ammonia in the culture solution 10 may be adsorbed to the adsorbent 36.

The adsorbent 36 is preferably coated with a resin such as polyvinyl alcohol; and a biological gel such as collagen, alginic acid, or gelatin, or the like. This successfully suppresses outflow of fine particles that may affect the cell 8 and the like, out from the adsorbent 36 into the culture solution. The adsorbent 36 may alternatively be formed, after kneading an adsorbent ingredient with ceramic binder, resin binder, biological gel or the like. This also makes it possible to suppress the outflow of fine particles. Examples of the ceramic binder include alumina binder and colloidal silica. Examples of the resin binder include polyvinyl alcohol, and carboxymethyl cellulose. Examples of the biological gel include collagen, alginic acid, and gelatin.

The embodiments of the present invention have been described in detail above. The above-described embodiments merely illustrate specific examples in carrying out the present invention. Contents of the embodiments do not limit the technical scope of the present invention, instead allowing numerous design changes such as modification, addition, and deletion of constituents, without departing from the spirit of the present invention specified in the claims. Any new embodiment with design change will have effects derived both from an embodiment and modification to be combined. In the above-described embodiments, all contents possibly subject to such design change have been emphasized with a notation stating "... of the present embodiment" or "in the present embodiment". Also any content without such notation is, however, acceptable for the design change. Free combination of the above constituents is also valid as an aspect of the present invention.

### [EXAMPLES]

Examples of the present invention will be explained below. Examples are merely illustrative, and by no means limit the present invention.

### Determination of Lactic Acid Concentration Influential to Cell Proliferation

Onto a 24-well plate (Corning cellBIND Surface: from Corning Incorporated), seeded were 1 × 10³ human pluripotent stem cells, to which a medium for pluripotent stem cell (StemFit: from Ajinomoto Co. Inc.) was added, and then cultured. To the medium before 24 hours from the start of culture, a culture substrate (iMatrix-511; from Nippi, Inc.) was added so as to adjust the concentration to 0.5 pg/ml, and also ROCK inhibitor (Y-27632: from FUJIFILM Wako Pure Chemical Corporation) was added so as to adjust the concentration to 10 µM. Meanwhile to the medium at 24 hours or after from the start of culture, lactic acid (FUJIFILM Wako Pure Chemical Corporation) was added so as to adjust the lactic acid concentration in various levels. The lactic acid concentration was varied among 0 mM, 2.5 mM, 5.0 mM, 7.5 mM, 10.0 mM and 15.0 mM.

As lactic acid is added, pH of the medium will decrease. Hence, the inventors prepared an experimental group with pH of the medium corrected to 7.2, and an uncorrected experimental group. In the uncorrected experimental group, the medium with a lactic acid concentration of 5.0 mM was found to have pH 6.9, the medium with a lactic acid concentration of 7.5 mM was found to have pH 6.8, medium with a lactic acid concentration of 10 mM was found to have pH 6.7, and the medium with a lactic acid concentration of 15 mM was fond to have pH 6.5.

The culture was continued four days while refreshing the medium every day (hence the culture period totals 5 days). After the elapse of 5 days, the number of cells in each well was measured by using TC20 full-automatic cell counter (from Bio-Rad Laboratories, Inc.), to calculate cell density. Results are illustrated in Fig. 5. Fig. 5 is a graph illustrating relation between the lactic acid concentration and the cell density.

As can be seen in Fig. 5, the cell density was found to sharply decrease at a lactic acid concentration of 7.5 mM or higher, irrespective of pH correction. The pH-corrected experimental group showed the cell density at a lactic acid concentration of 15 mM, reduced by half or more from the value at a lactic acid concentration of 0 mM. In contrast, the pH-uncorrected group showed the cell density at a lactic acid concentration of 10 mM or above, sharply reduced down below the cell density observed at a lactic acid concentration of 15 mM in the pH-corrected group. This confirms that lactic acid *per se* inhibits cell proliferation, and also inhibits cell proliferation by inducing pH reduction.

It was thus confirmed from the results that the cell proliferation is effectively suppressed from degrading, by keeping the lactic acid concentration to lower than 7.5 mM. It was also confirmed that the lactic acid-induced inhibition of cell proliferation may be suppressed more reliably, by keeping the lactic acid concentration to 5 mM or below.

### Determination of Lactic Acid Concentration Influential to Undifferentiated State of Cell

Onto a 24-well plate (Corning cellBIND Surface: from Corning Incorporated), seeded were 1 × 10³ human pluripotent stem cells, to which a medium for pluripotent stem cell (StemFit: from Ajinomoto Co. Inc.) was added, and then cultured. To the medium before 24 hours from the start of culture, a culture substrate (iMatrix-511; from Nippi, Inc.) was added so as to adjust the concentration to 0.5 pg/ml, and also ROCK inhibitor (Y-27632: from FUJIFILM Wako Pure Chemical Corporation) was added so as to adjust the concentration to 10 µM. Meanwhile to the medium at 24 hours or after from the start of culture, lactic acid (FUJIFILM Wako Pure Chemical Corporation) was added so as to adjust the lactic acid concentration in various levels. The lactic acid concentration was varied among 0 mM, 1.0 mM, 2.5 mM, 5.0 mM, 7.5 mM, 10.0 mM and 15.0 mM.

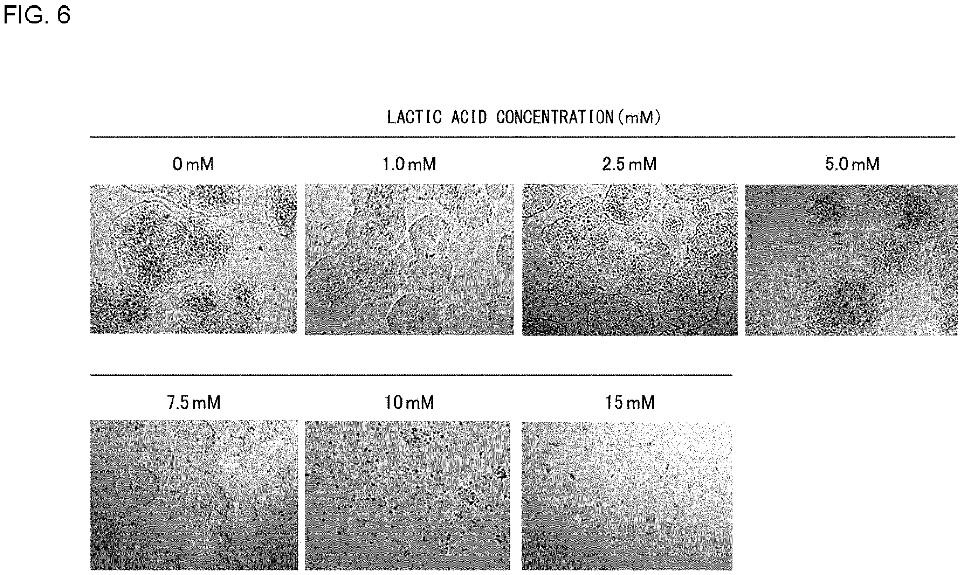

The culture was continued four days while refreshing the medium every day (hence the culture period totals 5 days). The individual cells on the 5th day of culture were observed under an optical microscope. Fig. 6 presents optical microphotographs of the cells cultured in the lactic acid-added medium. As can be confirmed from Fig. 6, colonies of the cells markedly decreased at a lactic acid concentration of 10 mM or higher, indicating inhibition of cell proliferation.

mRNA was then extracted from each cell on the 6th day of culture, using RNeasy Mini Kit (from QIAGEN N.V.), and then purified. Next, cDNA was synthesized from the thus purified mRNA, by using QuantiTect Reverse Transcription Kit (from QIAGEN N.V.). By using each cDNA as a template, and by using Thermal Cycler Dice Real Time System I (from Takara Bio Inc.), expression levels of Oct4, Nanog, T, Pax6 and Fox2 genes were measured by real-time PCR.

Oct4 and Nanog are undifferentiation markers indicating that the pluripotent stem cell retains its property. T, Pax6 and Fox2 are differentiation markers indicating that the pluripotent stem cell no longer retains its property, that is, the pluripotent stem cell has differentiated. More specifically, expression of T gene indicates that the cell has differentiated into mesoderm, expression of Pax6 gene indicates that the cell has differentiated into ectoderm, and expression of Fox2 indicates that the cell has differentiated into endoderm.

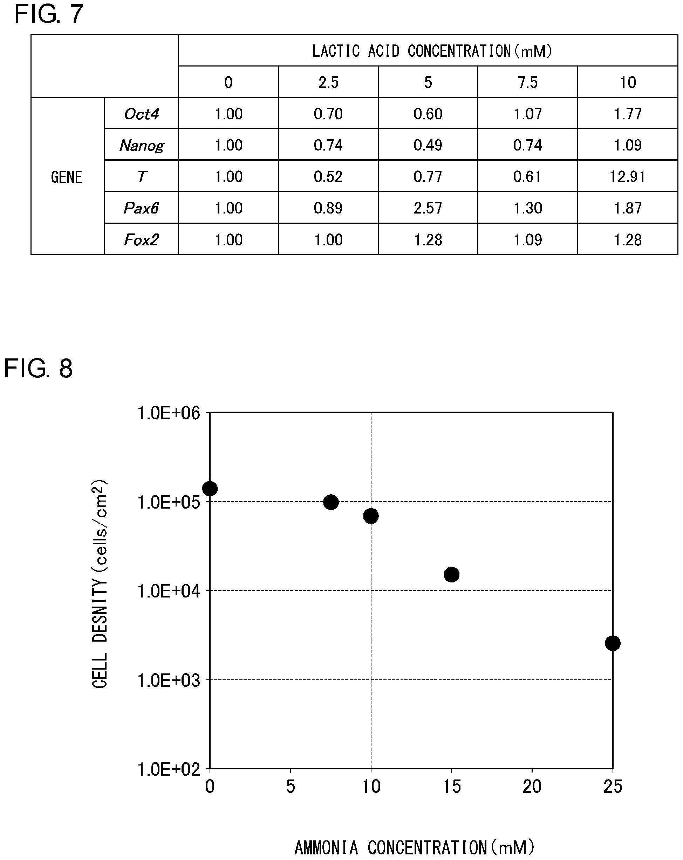

Proportion of expression levels of the individual genes at the individual concentration levels of lactic acid were calculated, on the basis of the expression level at a lactic acid concentration of 0 mM. Results are summarized in Fig. 7. Fig. 7 is a chart summarizing relation between the lactic acid concentration and the gene expression levels. As can be seen in Fig. 7, the expression level of T gene, which is a mesoderm differentiation marker, was found to increase at a lactic acid concentration of 10mM, to a level ten times or more of the level at a lactic acid concentration of 0 mM. This confirms that keeping of the lactic acid concentration at lower than 10 mM is effective to keep the cell in the undifferentiated state.

### Determination of Ammonia Concentration Influential to Cell Proliferation

Onto a 24-well plate (Corning cellBIND Surface: from Corning Incorporated), seeded were 1 × 10³ human pluripotent stem cells, to which a medium for pluripotent stem cell (StemFit: from Ajinomoto Co. Inc.) was added, and then cultured. To the medium before 24 hours from the start of culture, a culture substrate (iMatrix-511; from Nippi, Inc.) was added so as to adjust the concentration to 0.5 pg/ml, and also ROCK inhibitor (Y-27632: from FUJIFILM Wako Pure Chemical Corporation) was added so as to adjust the concentration to 10 µM. To the medium at 24 hours or after from the start of culture, ammonium chloride (FUJIFILM Wako Pure Chemical Corporation) was added so as to adjust the ammonia (ammonium ion) concentration to various levels. The ammonia concentration was varied among 0 mM, 7.5 mM, 10.0 mM, 15.0 mM and 25.0 mM.

The culture was continued four days while refreshing the medium every day (hence the culture period totals 5 days). After the elapse of 5 days, the number of cells in each well was measured by using TC20 full-automatic cell counter (from Bio-Rad Laboratories, Inc.), to calculate cell density. Results are summarized in Fig. 8. Fig. 8 is a graph illustrating relation between the ammonia concentration and the cell density.

As can be seen in Fig. 8, the cell density was found to sharply decrease at an ammonia concentration of 10.0 mM or higher. This confirms that keeping of the ammonia concentration at lower than 10 mM is effective to suppress inhibition of cell proliferation. It was also confirmed that the ammonia-induced inhibition of cell proliferation may be suppressed more reliably, by keeping the ammonia concentration at 7.5 mM or below.

### Determination of Ammonia Concentration Influential to Undifferentiated State of Cell

Onto a 24-well plate (Corning cellBIND Surface: from Corning Incorporated), seeded were 1 × 10³ human pluripotent stem cells, to which a medium for pluripotent stem cell (StemFit: from Ajinomoto Co. Inc.) was added, and then cultured. To the medium before 24 hours from the start of culture, a culture substrate (iMatrix-511; from Nippi, Inc.) was added so as to adjust the concentration to 0.5 µg/ml, and also ROCK inhibitor (Y-27632: from FUJIFILM Wako Pure Chemical Corporation) was added so as to adjust the concentration to 10 µM. To the medium at 24 hours or after from the start of culture, ammonium chloride (FUJIFILM Wako Pure Chemical Corporation) was added so as to adjust the ammonia concentration to various levels. The ammonia concentration was varied among 0 mM, 0.1 mM, 1.0 mM, 2.5 mM, 5.0 mM, 7.5 mM, 10.0 mM and 20.0 mM.

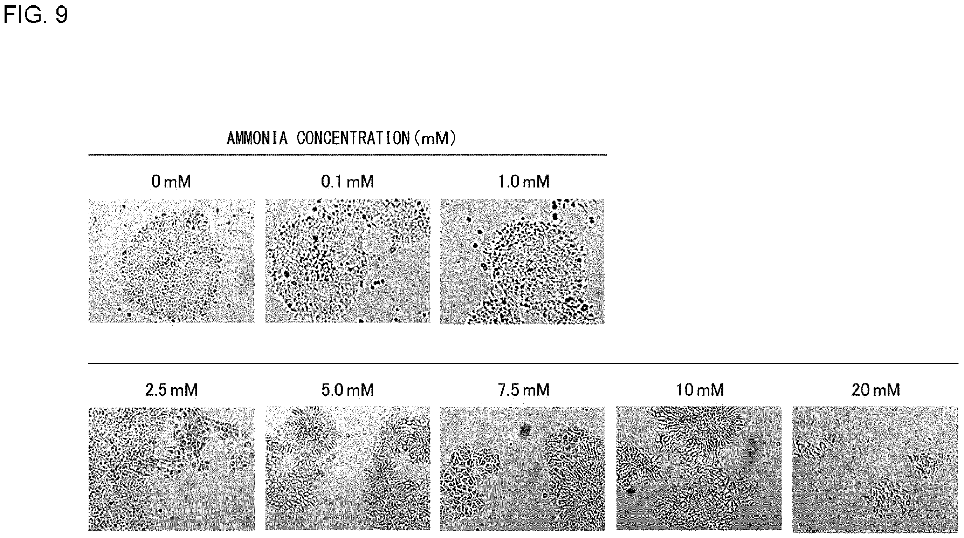

The culture was continued four days while refreshing the medium every day (hence the culture period totals 5 days). The individual cells on the 5th day of culture were observed under an optical microscope. Fig. 9 presents optical microphotographs of the cells cultured in the ammonia-added medium. As can be seen in Fig. 9, morphological changes of the cell were observed at an ammonia concentration of 2.5 mM or above.

mRNA was then extracted from each cell on the 6th day of culture, using RNeasy Mini Kit (from QIAGEN N.V.), and then purified. Next, cDNA was synthesized from the thus purified mRNA, by using QuantiTect Reverse Transcription Kit (from QIAGEN N.V.). By using each cDNA as a template, and by using Thermal Cycler Dice Real Time System I (from Takara Bio Inc.), expression levels of Oct4, Nanog, T, Pax6 and Fox2 genes were measured by real-time PCR.

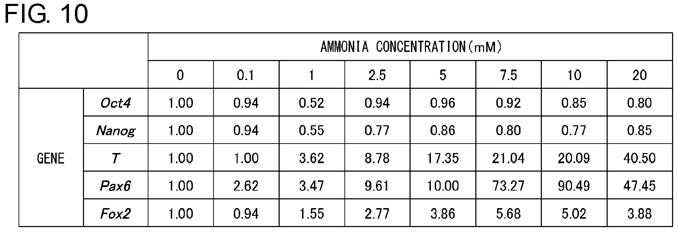

Proportion of expression levels of the individual genes at the individual concentration levels of ammonia were calculated, on the basis of the expression level at an ammonia concentration of 0 mM. Results are summarized in Fig. 10. Fig. 10 is a chart summarizing relation between the ammonia concentration and the gene expression levels. As can be seen in Fig. 10, the expression level of T gene, which is a mesoderm differentiation marker, and Pax6 gene, which is an ectoderm differentiation marker, were found to increase at an ammonia concentration of 2.5 mM, to a level nearly ten times as large as the level at an ammonia concentration of 0 mM. This confirms that keeping of the ammonia concentration at lower than 2.5 mM is effective to keep the cell in the undifferentiated state. It was also confirmed that the ammonia-induced promotion of cell differentiation may be suppressed more reliably, by keeping the ammonia concentration at 1 mM or below.

The findings confirm that the lactic acid concentration and the ammonia concentration in the medium are necessarily kept lower than 7.5 mM and lower than 2.5 mM, respectively, in order to allow the pluripotent stem cell to proliferate in the undifferentiated state.

### [INDUSTRIAL APPLICABILITY]

The present invention is applicable to the cell culture method and the cell culture apparatus.

### [REFERENCE SIGNS LIST]

1 cell culture apparatus, 2 culture vessel, 4 concentration sensor, 6 conditioner, 8 cell, 10 culture solution, 12 storage tank, 14 feed pump, 16 controller, 18 replenishing solution, 26 substance exchanger, 28 circulation pump, 30 substance exchange membrane, 32 ingredient conditioning solution, 36 adsorbent.

## Claims

1. A cell culture method comprising culturing a cell in a culture solution that satisfies at least one of condition (I) or condition (II) below:
condition (I): lactic acid concentration in the culture solution being lower than 7.5 mM;
and
condition (II): ammonia concentration in the culture solution being lower than 2.5 mM.

2. The cell culture method according to claim 1, wherein the cell is a pluripotent stem cell.

3. The cell culture method according to claim 1 or 2, wherein the lactic acid concentration in condition (I) is 5 mM or lower.

4. The cell culture method according to any one of claims 1 to 3, wherein the ammonia concentration in condition (II) is 1 mM or lower.

5. A cell culture apparatus comprising:
a culture vessel structured to house a cell and a culture solution; and
a conditioner structured to condition the culture solution so as to satisfy at least one of condition (I) or condition (II) below:
condition (I): lactic acid concentration in the culture solution being lower than 7.5 mM;
and
condition (II): ammonia concentration in the culture solution being lower than 2.5 mM.

6. The cell culture apparatus according to claim 5, wherein the conditioner comprises:
a storage tank structured to store a replenishing solution;
a feed pump structured to feed the replenishing solution from the storage tank to the culture vessel;
a concentration sensor structured to detect at least one of lactic acid concentration or ammonia concentration in the culture solution; and
a controller structured to drive the feed pump in response to result of detection by the concentration sensor.

7. The cell culture apparatus according to claim 5, wherein the conditioner comprises:
a substance exchanger structured to house a substance exchange membrane and an ingredient conditioning solution, and, upon feeding of the culture solution, allow substance exchange to proceed through the substance exchange membrane between the ingredient conditioning solution and the culture solution;
a circulation pump structured to circulate the culture solution between the culture vessel and the substance exchanger;
a concentration sensor structured to detect at least one of lactic acid concentration or ammonia concentration in the culture solution; and
a controller structured to drive the circulation pump in response to result of detection by the concentration sensor.

8. The cell culture apparatus according to claim 5, wherein the conditioner comprises an adsorbent structured to adsorb at least one of lactic acid or ammonia in the culture solution.
